# EUROPEAN PATENT APPLICATION

(11) **EP 4 382 595 A1**
(43) Date of publication of application: **12.06.2024**
(21) Application number: 22864619.6
(22) Date of filing: 31.08.2022
(51) Int. Cl.: C12M 1/04, B01D 53/14, C12M 1/00, C12M 1/107, C12N 1/20, C12P 1/04, C25B 3/03, C25B 3/26, C25B 15/08

(54) **BIOREACTOR AND CARBON DIOXIDE RECYCLING SYSTEM**

(30) Priority: 01.09.2021 JP 2021142234
(71) Applicant: Japan Agency for Marine-Earth Science and Technology, Yokosuka-shi Kanagawa 237-0061 (JP); Kanto Natural Gas Development Co., Ltd.,, Mobara-shi, Chiba 297-0026 (JP)
(72) Inventor: ISHII Shunichi, Yokosuka-shi, Kanagawa 237-0061 (JP); INAGAKI Fumio, Yokosuka-shi, Kanagawa 237-0061 (JP); IMACHI Hiroyuki, Yokosuka-shi, Kanagawa 237-0061 (JP); KAWANO Kenjiro, Mobara-shi, Chiba 297-0026 (JP); MURAI Daisuke, Mobara-shi, Chiba 297-0026 (JP)
(74) Representative: Michalski Hüttermann & Partner Patentanwälte mbB
(86) International application number: PCT/JP2022/032748
(87) International publication number: WO 2023/033037

(57) **Abstract**

The present invention relates to a bioreactor including: a container having an inlet and an outlet for a fluid; and a plurality of plate-shaped electrodes disposed in the container, in which a flow path in one direction from the inlet to the outlet is formed inside the container, and the flow path is formed such that opposing flows are adjacent to each other with the plate-shaped electrodes serving as partition walls interposed therebetween.

## Description

### Technical Field

The present invention relates to a bioreactor and a carbon dioxide recycling system.

### Background Art

Carbon dioxide is one of powerful greenhouse gases. Reducing the emission amount of carbon dioxide into the atmosphere is an important issue for global warming countermeasures. As a method for reducing the emission amount of carbon dioxide into the atmosphere, it has been attempted to recover carbon dioxide generated by combustion of fossil fuel or the like, convert the carbon dioxide into a resource substance using an electrochemical reaction by a microorganism, and reuse the resource substance.

For example, Patent Literature 1 discloses a method for converting carbon dioxide into methane by at least one of a reaction of the following Formula (1) and a reaction of the following Formula (3) accompanied by the following Formula (2) using methane bacteria.

CO₂ + 8H⁺ + 8e⁻ → CH₄ + 2H₂O Formula (1)

2H⁺ + 2e⁻ → H₂ Formula (2)

CO₂ + 4H₂ → CH₄ + 2H₂O Formula (3)

### Citation List

### Patent Literature

Patent Literature 1: Japanese Unexamined Patent Publication No. 2012-152137

### Summary of Invention

### Technical Problem

It is expected that a low-cost carbon dioxide reusing (recycling) process can be constructed by converting carbon dioxide into a resource substance using an electrochemical reaction by a microorganism. However, in order to put this into practical use, it is necessary to further improve the conversion efficiency.

An object of the present invention is to provide a bioreactor in which the conversion efficiency of a reaction of electrochemically converting carbon dioxide into a resource substance by microorganism is enhanced, whereby carbon dioxide can be converted into a resource substance at low cost. Another object of the present invention is to provide a carbon dioxide recycling system using the bioreactor.

### Solution to Problem

The present invention relates to a bioreactor including: a container having an inlet and an outlet for a fluid; and a plurality of plate-shaped electrodes disposed in the container, in which a flow path in one direction from the inlet to the outlet is formed inside the container, and the flow path is formed such that opposing flows are adjacent to each other with the plate-shaped electrodes serving as partition walls interposed therebetween.

Since the bioreactor according to the present invention includes the above configuration, cations (for example, hydrogen ions) produced at an anode electrode are transferred to a cathode electrode by the flow of the fluid, and the fluid flows to be in contact with both surfaces of the electrode, thereby enhancing the contact efficiency between the fluid and the electrode, and reducing the solution resistance with respect to transport of the cations between the electrodes. As a result, a current value per electrode area increases, and the efficiency of conversion from carbon dioxide to a resource substance (electrochemical reaction by a microorganism) is improved. Therefore, carbon dioxide can be converted into a resource substance at low cost by performing conversion from carbon dioxide into a resource substance (electrochemical reaction by a microorganism) using the bioreactor according to the present invention.

In the bioreactor, a distance between adjacent plate-shaped electrodes is preferably 2 cm or less. As a result, since the solution resistance with respect to transport of the cations between the electrodes can be further reduced, a current value per electrode area increases, and the efficiency of conversion from carbon dioxide to a resource substance (electrochemical reaction by a microorganism) is further improved.

In the bioreactor, an anode electrode and a cathode electrode are preferably alternately arranged in a flow path direction. As a result, the solution resistance with respect to transport of the cations between the electrodes can be further reduced.

The bioreactor may further include a voltage application device connected to the plurality of plate-shaped electrodes.

The present invention also relates to a carbon dioxide recycling system including: a storage unit storing anaerobic groundwater; a reaction unit converting carbon dioxide contained in the anaerobic groundwater into a resource substance by an electrochemical reaction by a microorganism; and a transfer unit transferring the anaerobic groundwater from the storage unit to the reaction unit, in which the reaction unit has the above-described bioreactor according to the present invention.

Since the recycling system according to the present invention includes the bioreactor according to the present invention in the reaction unit, the efficiency of conversion from carbon dioxide to a resource substance (electrochemical reaction by a microorganism) is improved, and carbon dioxide can be converted into a resource substance at low cost.

In the recycling system, the transfer unit preferably has a dissolving means for further dissolving carbon dioxide in the anaerobic groundwater. In the bioreactor according to the present invention, the amount of carbon dioxide (dissolved carbon dioxide concentration) contained in anaerobic groundwater is increased, and accordingly, the production rate of a resource substance is remarkably improved. Therefore, by further dissolving carbon dioxide in the anaerobic groundwater in the transfer unit, the production rate of a resource substance in the reaction unit is remarkably improved.

### Advantageous Effects of Invention

According to the present invention, it is possible to provide a bioreactor in which the conversion efficiency of a reaction of electrochemically converting carbon dioxide into a resource substance by microorganism is enhanced, whereby carbon dioxide can be converted into a resource substance at low cost. According to the present invention, it is possible to provide a carbon dioxide recycling system using the bioreactor.

### Brief Description of Drawings

FIG. 1 is a perspective view of a bioreactor according to an embodiment.
FIG. 2 is a front view of the bioreactor according to the embodiment.
FIG. 3 is a side view of the bioreactor according to the embodiment.
FIG. 4 is a plan view of the bioreactor according to the embodiment.
FIG. 5 is a schematic diagram of a carbon dioxide recycling system according to an embodiment.
FIG. 6(A) is a graph showing a relationship between an applied voltage and a current value. FIG. 6(B) is a graph showing a relationship between an applied voltage and a current value per unit area of an electrode.
FIG. 7(A) is a graph showing a relationship between a flow rate of anaerobic groundwater and a current value. FIG. 7(B) is a graph showing a relationship between a flow rate of anaerobic groundwater and a current value per unit area of an electrode.
FIG. 8 is a graph showing a relationship between a method of supplying anaerobic groundwater and an electrogenic methane-producing rate.
FIG. 9 is a graph showing a relationship between the presence or absence of a flow of anaerobic groundwater and an electrogenic methane-producing rate.
FIG. 10 is a front view of a bioreactor of a reference example.
FIG. 11 is a side view of the bioreactor of the reference example.
FIG. 12 is a plan view of the bioreactor of the reference example.

### Description of Embodiments

Hereinafter, modes for carrying out the present invention will be described in detail with reference to the drawings as necessary. However, the present invention is not limited to the following embodiments.

A bioreactor according to the present embodiment includes a container having an inlet and an outlet for a fluid, and a plurality of plate-shaped electrodes disposed inside the container. Here, a flow path in one direction from the inlet to the outlet is formed inside the container, and the flow path is formed such that opposing flows are adjacent to each other with the plate-shaped electrodes serving as partition walls interposed therebetween.

When the fluid is introduced from the inlet of the bioreactor, a flow of the fluid is generated along the flow path formed inside the container. At this time, since the flow path is formed as a flow path in one direction from the inlet to the outlet of the container, the fluid flows through the flow path in one way. Since the flow path is formed such that opposing flows are adjacent to each other with the plate-shaped electrodes serving as partition walls interposed therebetween, cations (for example, hydrogen ions) produced at an anode electrode are transferred to a cathode electrode by the flow of the fluid, and the fluid flows to be in contact with both surfaces of the electrode, thereby enhancing the contact efficiency between the fluid and the electrode, and reducing the solution resistance with respect to transport of the cations between the electrodes.

The number of plate-shaped electrodes disposed inside the bioreactor may be at least two because the plate-shaped electrodes need to function as an anode electrode and a cathode electrode. From the viewpoint of further increasing a current value per electrode area, the number of plate-shaped electrodes is preferably larger, and may be, for example, 4 or more, 8 or more, 12 or more, or 16 or more. The upper limit of the number of plate-shaped electrodes is not particularly limited, and may be, for example, 32 or less, 28 or less, 24 or less, or 20 or less.

The plate-shaped electrodes disposed inside the bioreactor function as an anode electrode and a cathode electrode by being connected to a voltage application device (for example, an external power supply). At this time, the number of anode electrodes and the number of cathode electrodes do not necessarily need to match each other. The arrangement of the anode electrode and the cathode electrode may be arbitrarily set, but it is preferable that the anode electrode and the cathode electrode are alternately arranged in a flow path direction (a flow direction of the fluid) from the inlet to the outlet from the viewpoint of further reducing the solution resistance with respect to transport of the cations between the electrodes.

The flow path formed inside the bioreactor is formed such that opposing flows are adjacent to each other with the plate-shaped electrodes serving as partition walls interposed therebetween. It is preferable that the flow path is formed such that opposing flows are adjacent to each other with each of the plate-shaped electrodes serving as a partition wall interposed therebetween, so that a plurality of flow paths folded back at one end of each plate-shaped electrode are arranged to be adjacent to each other. As a result, the contact efficiency between the fluid and the electrode is further enhanced, and the solution resistance with respect to transport of the cations between the electrodes can be further reduced.

The distance between adjacent plate-shaped electrodes (distance in an out-of-plane direction of the plate-shaped electrodes) is preferably 2 cm or less, more preferably 1.5 cm or less, still more preferably 1 cm or less. As a result, the solution resistance with respect to transport of the cations between the electrodes can be further reduced.

The plate-shaped electrode may have a member supporting the shape, but it is preferable that at least a part of the electrode surface is exposed and it is more preferable that the entire electrode surface is exposed in order to bring the fluid into direct contact with the electrode.

The plate-shaped electrode may be an electrode formed in a plate shape. The electrode material is not particularly limited as long as it functions as an electrode, and examples thereof include carbon, stainless steel, and titanium. In a case where the plate-shaped electrode has a member supporting the shape, the member is preferably formed of an insulating material, and specific examples thereof include polystyrene, vinyl chloride, and an acrylic plate.

The container constituting the bioreactor is preferably formed of an insulating material, and specific examples thereof include vinyl chloride and an acrylic resin. The shape of the container is not particularly limited, but the container preferably has a rectangular parallelepiped shape because the plate-shaped electrodes can be densely arranged, whereby the contact efficiency between the fluid and the electrode can be enhanced.

In a bioreactor according to an embodiment, a plate-shaped electrode disposed to be in contact with a bottom surface and a first side wall inside a container and a plate-shaped electrode disposed to be in contact with the bottom surface and a second side wall facing the first side wall inside the container are alternately arranged, so that a flow path in one direction from an inlet to an outlet is formed inside the container, and the flow path is formed such that opposing flows are adjacent to each other with the plate-shaped electrodes serving as partition walls interposed therebetween.

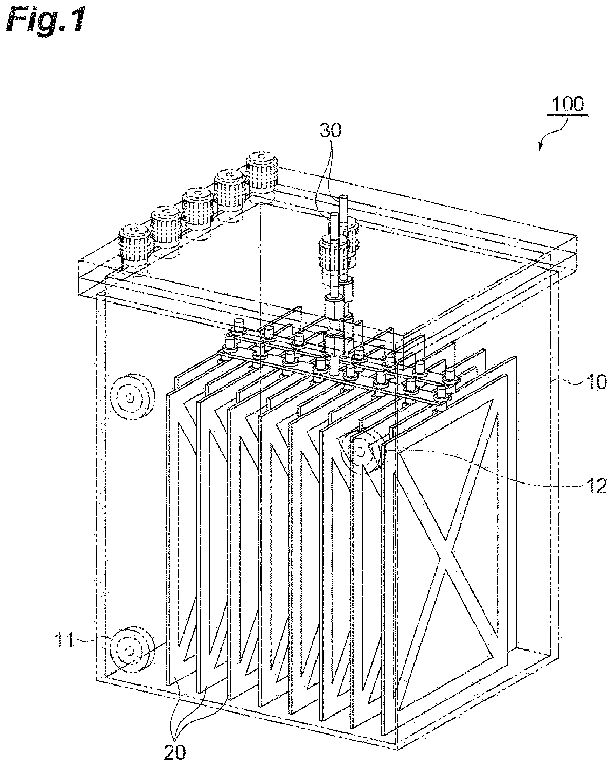

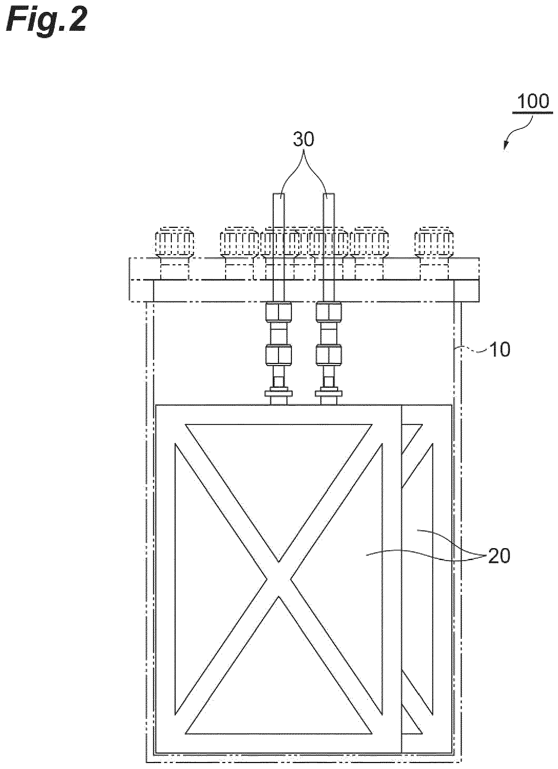

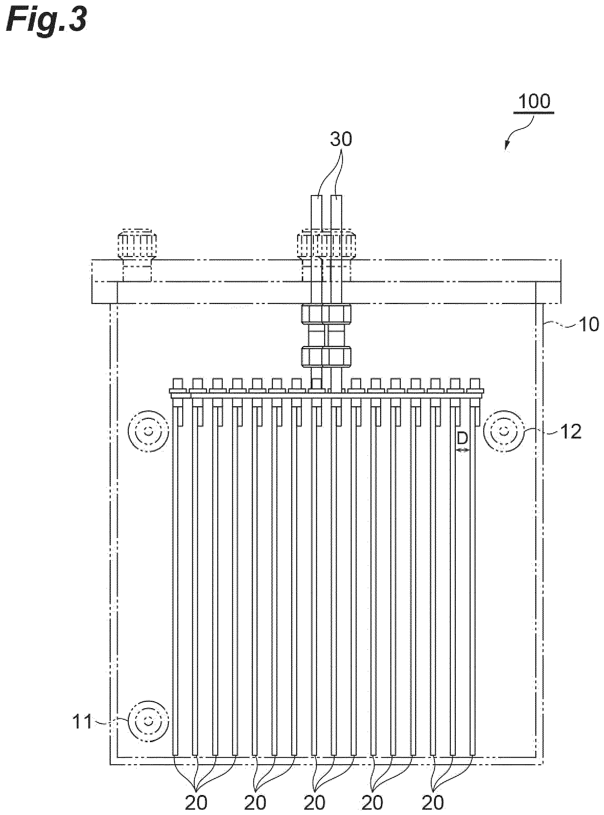

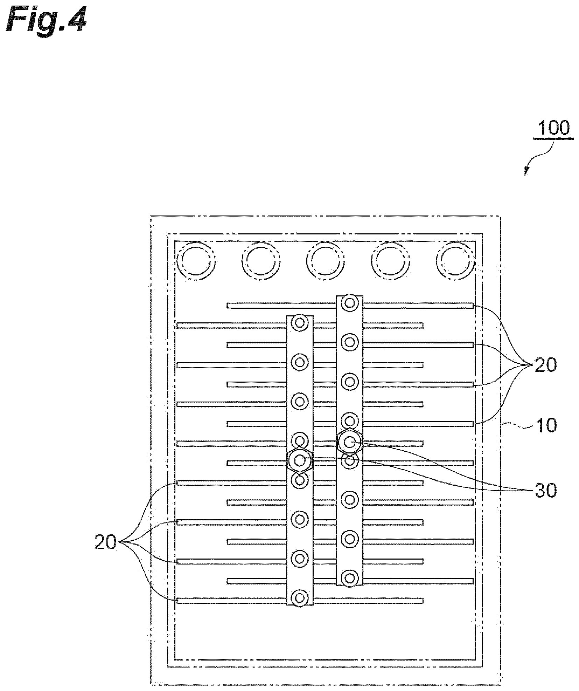

FIG. 1 is a perspective view of a bioreactor according to an embodiment. FIG. 2 is a front view of the bioreactor according to the embodiment. FIG. 3 is a side view of the bioreactor according to the embodiment. FIG. 4 is a plan view of the bioreactor according to the embodiment. A bioreactor 100 illustrated in FIGS. 1 to 4 includes a container 10 having a rectangular parallelepiped shape, and a plurality of plate-shaped electrodes 20 disposed inside the container 10. In the container 10 having a rectangular parallelepiped shape, an inlet 11 for introducing a fluid (for example, anaerobic groundwater) into the inside of the container 10 and an outlet 12 for discharging the fluid to the outside of the container 10. The plate-shaped electrodes 20 function as an anode electrode and a cathode electrode by being connected to a voltage application device (not illustrated) via a conductive wire 30. In the bioreactor 100 illustrated in FIGS. 1 to 4 is configured such that the plate-shaped electrodes 20 are connected to a voltage application device (not illustrated) via the conductive wire 30 so as to alternately arrange the anode electrode and the cathode electrode. A distance D between the plate-shaped electrodes 20 of the bioreactor 100 (distance in an out-of-plane direction of the plate-shaped electrodes 20) is, for example, 1 cm.

In the bioreactor 100, the plate-shaped electrode 20 disposed to be in contact with a bottom surface and a first side wall inside the container 10 and the plate-shaped electrode 20 disposed to be in contact with the bottom surface and a second side wall facing the first side wall inside the container 10 are alternately arranged, so that a flow path in one direction from the inlet 11 to the outlet 12 is formed inside the container 10. Note that, in the bioreactor 100, since the outlet 12 is formed at a position lower than the top of the plate-shaped electrode 20, the fluid does not fluid beyond the plate-shaped electrode 20. This flow path is formed such that opposing flows of the fluid are adjacent to each other with the plate-shaped electrodes 20 serving as partition walls interposed therebetween.

The bioreactor according to the present embodiment can be used to introduce anaerobic water, carbon dioxide, and a microorganism having an ability to convert carbon dioxide into a resource substance by an electrochemical reaction (hereinafter, also referred to as "electron-utilizing microorganism") into the inside the bioreactor so as to perform a reaction in which carbon dioxide is converted into a resource substance using an electrochemical reaction by an electron-utilizing microorganism.

The anaerobic water may be oxygen-depleted water. The anaerobic water is in a reductive state due to lack of oxygen, and can efficiently release electrons (e⁻) at the anode electrode. The anaerobic water may be, for example, naturally occurring anaerobic water (anaerobic groundwater) such as water existing in an anaerobic underground aquifer or brine water staying in an oil field or a gas field, or may be anaerobic water obtained by performing anaerobic treatment on water. Examples of the anaerobic treatment method for obtaining anaerobic water include a method of bubbling water with an inert gas to remove dissolved oxygen and a method of adding a reducing agent such as sodium sulfide or titanium chloride to water to remove dissolved oxygen. The anaerobic groundwater can be preferably used as a fluid to be introduced into the bioreactor according to the present embodiment because carbon dioxide is dissolved in anaerobic groundwater and a subterranean microorganism is contained (for example, methanogen is contained in brine water staying in an oil field or a gas field).

As carbon dioxide, carbon dioxide (dissolved carbon dioxide) originally contained in the anaerobic water may be used, or carbon dioxide dissolved by blowing carbon dioxide gas into the anaerobic water or the like may be used. Examples of the method of dissolving carbon dioxide in anaerobic water include a method of blowing carbon dioxide gas into anaerobic water, a method of adding dry ice to anaerobic water, and a method of spraying anaerobic water in a carbon dioxide gas atmosphere (for example, a method using a CO₂ high-concentration dissolving apparatus (trade name: High-Concentration Oxygen Dissolving Apparatus, manufactured by TOMOE SHOKAI Co., LTD.)). Note that, carbon dioxide may be further dissolved in anaerobic water containing dissolved carbon dioxide.

As the electron-utilizing microorganism, a microorganism having an ability to convert carbon dioxide into a resource substance by an electrochemical reaction can be used without particular limitation. Examples of the electron-utilizing microorganism include a microorganism having an ability to convert carbon dioxide into a hydrocarbon such as methane by an electrochemical reaction (such as methanogen), a microorganism having an ability to convert carbon dioxide into an organic acid such as acetic acid by an electrochemical reaction (such as acetogenic bacterium), and a microorganism having an ability to convert carbon dioxide into a biodegradable polymer such as polyhydroxyalkanoic acid (PHA) by an electrochemical reaction (such as PHA-forming bacterium).

More specific examples of the electron-utilizing microorganism include a methanogen belonging to the class Methanobacteria, a methanogen belonging to the class Methanomicrobia, a methanogen belonging to the class Methanococci, a methanogen belonging to the class Methanopyri, an acetogenic bacterium belonging to the class Clostridia, an acetogenic bacterium belonging to the class Negativicutes, and a PHA-forming bacterium belonging to the class Betaproteobacteria.

The method of introducing anaerobic water, carbon dioxide, an electron-utilizing microorganism into the inside of the bioreactor is not particularly limited. Specifically, for example, a fluid containing anaerobic water, carbon dioxide, an electron-utilizing microorganism may be prepared, and the fluid may be introduced into the bioreactor, or a cathode electrode on which an electron-utilizing microorganism is immobilized may be disposed in the bioreactor in advance, and a fluid containing anaerobic water and carbon dioxide may be introduced into the reactor, and these methods may be used in combination.

By introducing anaerobic water, carbon dioxide, and an electron-utilizing microorganism into the bioreactor, electrons (e⁻) are first released from the anaerobic water at the anode electrode, and counterions (cations; for example, hydrogen ions) are generated. The cations then flow along the flow path with the fluid and reach the cathode electrode. The electrons reach the cathode electrode via an external power supply (voltage application device). In the cathode electrode, the electron-utilizing microorganism performs a reaction of converting carbon dioxide into a resource substance using electrons (and cations as necessary). For example, in a case where the resource substance is methane, the conversion reaction is performed by a reaction represented by CO₂ + 8H⁺ + 8e⁻ → CH₄ + 2H₂O. The voltage applied by the voltage application device may be appropriately set within a range of, for example, 0.1 to 5.0 V The voltage applied by the voltage application device may be in a range of 0.2 to 3.0 V, 0.3 to 2.0 V, 0.4 to 1.5 V, or 0.5 to 1.2 V The generated resource substance may be recovered, for example, as gas from the gas phase portion of the bioreactor, or may be recovered from the fluid discharged from the outlet.

The bioreactor according to the present embodiment can be used by being incorporated in a carbon dioxide recycling system.

A carbon dioxide recycling system according to the present embodiment includes: at least a storage unit storing anaerobic groundwater; a reaction unit converting carbon dioxide contained in the anaerobic groundwater into a resource substance by an electrochemical reaction by a microorganism; and a transfer unit transferring the anaerobic groundwater from the storage unit to the reaction unit. Here, the reaction unit has the bioreactor according to the present invention. Since the recycling system according to the present embodiment includes the bioreactor according to the present invention in the reaction unit, the efficiency of conversion from carbon dioxide to a resource substance (electrochemical reaction by a microorganism) is improved, and carbon dioxide can be converted into a resource substance at low cost.

In the carbon dioxide recycling system according to the present embodiment, the transfer unit may have a dissolving means for further dissolving carbon dioxide in the anaerobic groundwater. By further dissolving carbon dioxide in the anaerobic groundwater in the transfer unit, the production rate of a resource substance in the reaction unit is remarkably improved.

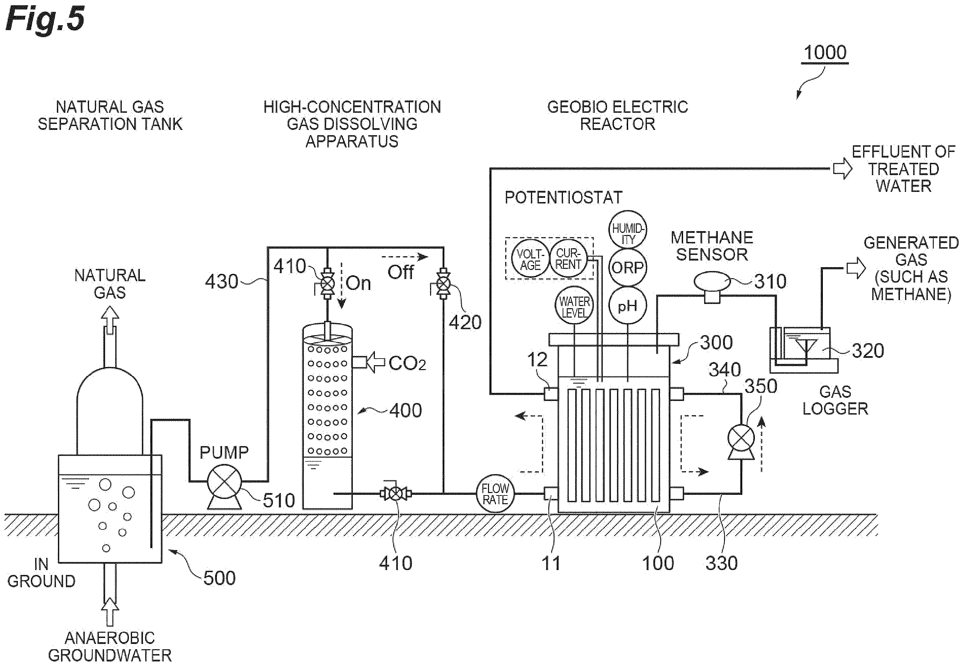

FIG. 5 is a schematic diagram of a carbon dioxide recycling system according to an embodiment. A carbon dioxide recycling system 1000 illustrated in FIG. 5 includes: a storage unit 500 storing anaerobic groundwater; a reaction unit 300 converting carbon dioxide contained in the anaerobic groundwater into a resource substance by an electrochemical reaction by a microorganism; and a transfer unit transferring the anaerobic groundwater from the storage unit to the reaction unit.

The storage unit 500 may be configured as, for example, a natural gas separation tank. In this case, the anaerobic groundwater drawn from the ground is temporarily stored in the natural gas separation tank, and the natural gas is separated in the natural gas separation tank. The anaerobic groundwater from which the natural gas has been separated is transferred to the reaction unit 300 via the transfer unit.

The transfer unit has a pump 510 and a conduit 430 for feeding anaerobic groundwater from the storage unit 500, and the conduit 430 is connected to the inlet 11 of the bioreactor 100. In the carbon dioxide recycling system 1000, the transfer unit has a dissolving means 400 for further dissolving carbon dioxide in the anaerobic groundwater. In conjunction with the opening and closing (On/Off) of an electromagnetic valve 410 and an electromagnetic valve 420 provided in the middle of the conduit 430, the anaerobic groundwater transferred by the conduit 430 is guided to the dissolving means 400, and the treatment of further dissolving carbon dioxide is performed by dissolving means 400. The dissolving means 400 may be, for example, a high-concentration gas dissolving apparatus (for example, CO₂ high-concentration dissolving apparatus (trade name: High-Concentration Oxygen Dissolving Apparatus, manufactured by TOMOE SHOKAI Co., LTD.)), but is not limited thereto, and may be configured as an apparatus for blowing carbon dioxide gas into anaerobic groundwater or an apparatus for adding dry ice to anaerobic groundwater.

The reaction unit 300 has the bioreactor 100. The bioreactor 100 is configured as the above-described bioreactor according to the present invention. The bioreactor 100 may be operated to circulate the anaerobic groundwater inside the bioreactor 100 via a conduit 330, a pump 350, and a conduit 340. The bioreactor 100 may be equipped with a water level gauge, a pH sensor, a hygrometer, a redox potential (ORP) meter, a potentiostat, and the like, as necessary, to monitor a reaction. The reaction unit 300 may further include a means for recovering the generated resource substance (for example, methane). In a case where a resource substance to be generated is methane, for example, a product gas (methane gas) may be recovered via a conduit for recovering the gas from the upper portion of the bioreactor 100. At this time, a methane sensor 310 or a gas logger 320 for monitoring the methane production amount may be provided in the middle of the conduit. The reaction unit 300 may be configured as, for example, a geobioelectric reactor. The external power supply (voltage application device) to be connected to the bioreactor 100 may be, for example, a power supply derived from renewable energy (power generated by solar power generation, wind power generation, marine power generation, hydroelectric power generation, or the like) or night standby power.

### Examples

Hereinafter, the present invention will be more specifically described based on Examples. However, the present invention is not limited to the following Examples. In the following Examples, groundwater (brine water) drawn from a natural gas well (Minami-Kanto Gas Field) was used as anaerobic groundwater. Anaerobic groundwater includes a subterranean microbial community (for example, methanogen). The dissolved carbon dioxide concentration in the anaerobic groundwater was 28.5 mM.

### [Test Example 1: Measurement of Current Value When Voltage Applied to Bioreactor Is Changed]

Using the bioreactor (reactor capacity: 10 L, anaerobic groundwater capacity: 8 L, plate-shaped electrode size: 9.4 cm in width × 23 cm in length, nine cathode electrodes, nine anode electrodes, distance between adjacent plate-shaped electrodes: 1 cm) of the example having the configuration illustrated in FIGS. 1 to 4, the current value was measured when the applied voltage was changed. In the bioreactor of the example is configured such that the plate-shaped electrodes are connected to a voltage application device so as to alternately arrange the cathode electrode and the anode electrode. Hereinafter, the bioreactor of the example is also referred to as "comb-type 18-electrode bioreactor".

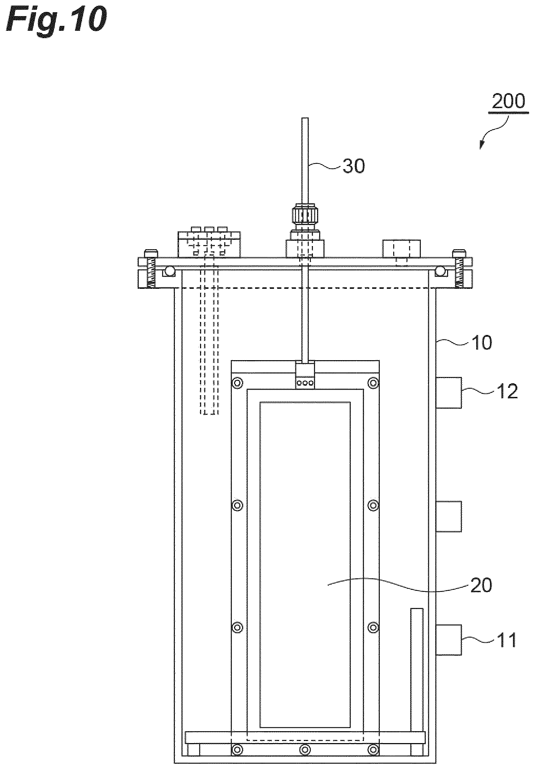

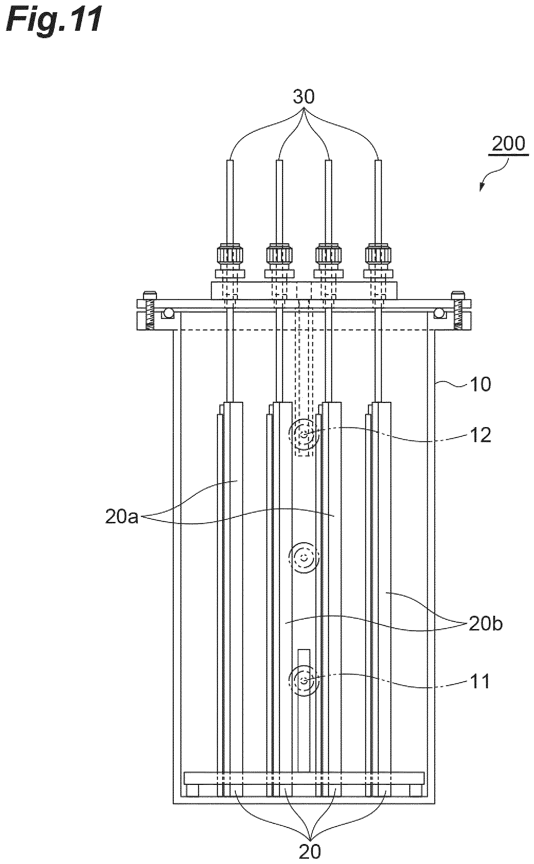

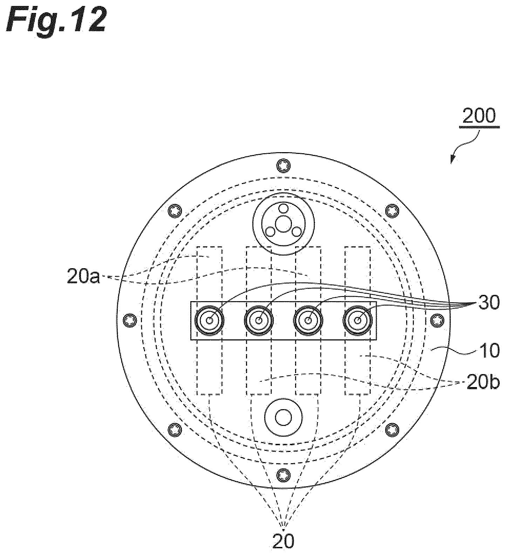

For comparison, using the bioreactor (reactor capacity: 10 L, anaerobic groundwater capacity: 8 L, plate-shaped electrode size: 9.5 cm in width × 29 cm in length, two cathode electrodes, two anode electrodes, distance between adjacent plate-shaped electrodes: 4 cm) of the reference example having the configuration illustrated in FIGS. 10 to 12, the same measurement was performed. FIG. 10 is a front view of a bioreactor of a reference example. FIG. 11 is a side view of the bioreactor of the reference example. FIG. 12 is a plan view of the bioreactor of the reference example. A bioreactor 200 illustrated in FIGS. 10 to 12 includes a container 10 having a cylindrical shape, and four plate-shaped electrodes 20 disposed inside the container 10. In the bioreactor 200 is configured such that the plate-shaped electrodes 20 are connected to a voltage application device (not illustrated) via a conductive wire 30 so as to alternately arrange a cathode electrode 20a and an anode electrode 20b. In the container 10 having a cylindrical shape, an inlet 11 for introducing anaerobic groundwater into the inside of the container and an outlet 12 for discharging the anaerobic groundwater to the outside of the container. In the bioreactor 200 illustrated in FIGS. 10 to 12, the anaerobic groundwater introduced from the inlet 11 can freely move back and forth between the plate-shaped electrodes 20 and between the plate-shaped electrodes 20 and the inner wall of the container 10. Hereinafter, the bioreactor of the reference example is also referred to as "cylindrical 4-electrode bioreactor".

Anaerobic groundwater was introduced into each of the comb-type 18-electrode bioreactor and the cylindrical 4-electrode bioreactor and maintained at a flow rate of 2 L/min. The anaerobic groundwater capacity inside the container in a steady state is 8 L in each case. To the anaerobic groundwater to be introduced into the bioreactor, acetic acid was added to have a concentration of 10 mM. In a steady state, a current value when a voltage was applied in a range of 0 V to 0.6 V by a voltage application device (external power supply) was measured.

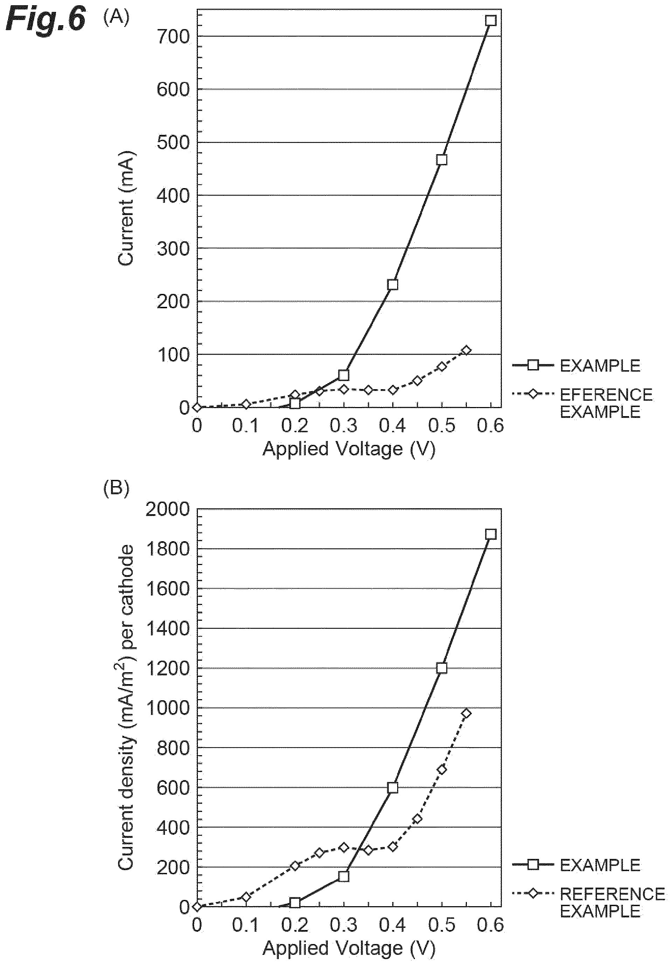

The results are shown in FIG. 6. FIG. 6(A) is a graph showing a relationship between an applied voltage and a current value. FIG. 6(B) is a graph showing a relationship between an applied voltage and a current value per unit area of an electrode. The comb-type 18-electrode bioreactor could obtain a larger current value than the cylindrical 4-electrode bioreactor (FIG. 6(A)), and also had a higher current value per unit area of the electrode (FIG. 6(B)). As the current value is larger, the electrochemical reaction by a microorganism can be efficiently performed. In the comb-type 18-electrode bioreactor, as the applied voltage is increased, the current value also increases in a straightforward manner, and it was presumed that no unnecessary side reaction occurred. This is also considered to be the reason why a larger current value was obtained than in the cylindrical 4-electrode bioreactor.

### [Test Example 2: Measurement of Current Value When Flow Rate of Anaerobic Groundwater Is Changed]

Using the comb-type 18-electrode bioreactor and the cylindrical 4-electrode bioreactor described in Test Example 1, the current value was measured when the flow rate of anaerobic groundwater was changed.

Anaerobic groundwater was introduced into each of the comb-type 18-electrode bioreactor and the cylindrical 4-electrode bioreactor and maintained at a specific flow rate (within a range of 0 to 3 L/min). The anaerobic groundwater capacity inside the container in a steady state is 8 L in each case. To the anaerobic groundwater to be introduced into the bioreactor, acetic acid was added to have a concentration of 10 mM. In a steady state, a current value when a voltage of 0.6 V (600 mV) was applied by a voltage application device (external power supply) was measured.

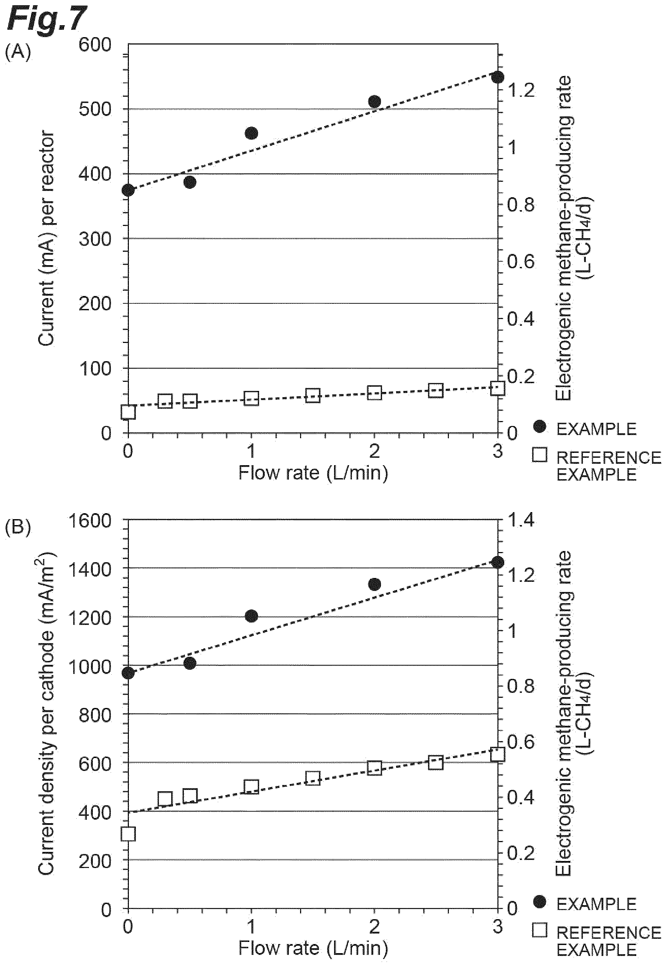

The results are shown in FIG. 7. FIG. 7(A) is a graph showing a relationship between a flow rate of anaerobic groundwater and a current value. FIG. 7(B) is a graph showing a relationship between a flow rate of anaerobic groundwater and a current value per unit area of an electrode. The comb-type 18-electrode bioreactor could obtain a larger current value than the cylindrical 4-electrode bioreactor at any flow rate measured (FIG. 7(A)), and also had a higher current value per unit area of the electrode (FIG. 7(B)). As the current value is larger, the electrochemical reaction by a microorganism can be efficiently performed. It has been confirmed that the current value increases in proportion to the flow rate of anaerobic groundwater. This is considered to be because, as the flow rate of anaerobic groundwater increases, the solution resistance with respect to transport of the cations between the electrodes is further reduced.

### [Test Example 3: Measurement of Electrogenic Methane-Producing Rate When Method of Supplying Anaerobic Groundwater Is Changed]

Using the comb-type 18-electrode bioreactor described in Test Example 1, the electrogenic methane-producing rate was measured when the method of supplying anaerobic groundwater was changed.

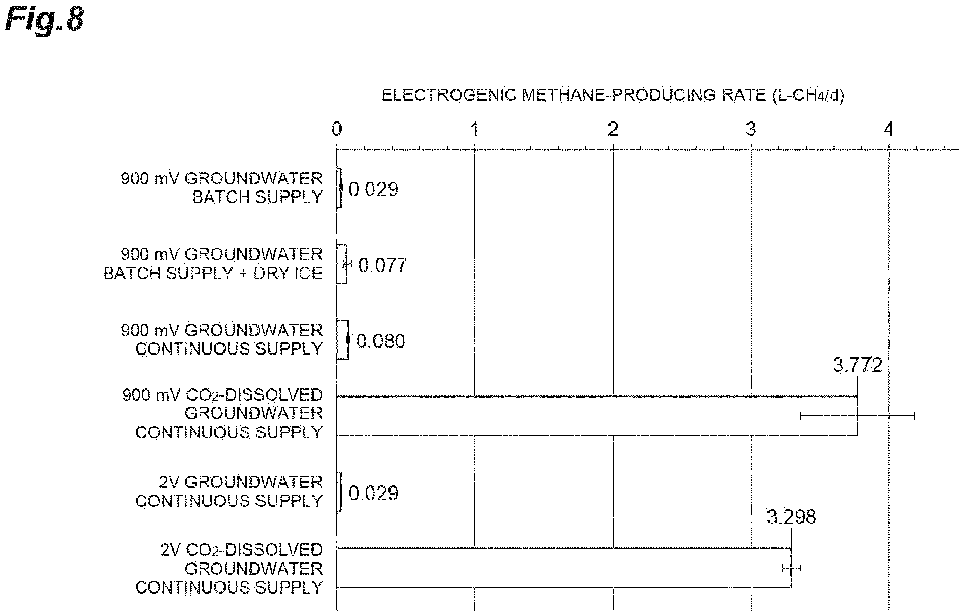

FIG. 8 is a graph showing a relationship between a method of supplying anaerobic groundwater and an electrogenic methane-producing rate. Details of each condition in FIG. 8 are as follows.

### <Test 1: 900 mV Groundwater Batch Supply>

Anaerobic groundwater was introduced into the comb-type 18-electrode bioreactor, the flow of anaerobic groundwater was set as internal circulation (flow rate: 2 L/min), and the electrogenic methane-producing rate was measured. The anaerobic groundwater capacity inside the container is 8 L. A voltage of 0.9 V (900 mV) was applied by a voltage application device (external power supply), and the amount of generated gas (methane) was measured with a methane sensor (BCP-CH4, manufactured by BlueSens gas sensor GmbH) and a gas logger (Milligas counter, manufactured by Japan Flow Controls Co., Ltd.).

### <Test 2: 900 mV Groundwater Batch Supply + Dry Ice>

Anaerobic groundwater was introduced into the comb-type 18-electrode bioreactor, the flow of anaerobic groundwater was set as internal circulation (flow rate: 2 L/min), and the electrogenic methane-producing rate was measured. The anaerobic groundwater capacity inside the container is 8 L. To anaerobic groundwater to be introduced into the bioreactor, about 1 g/L of dry ice was added (as a result, the pH of the anaerobic groundwater was changed from 8 to 6.8). A voltage of 0.9 V (900 mV) was applied by a voltage application device (external power supply), and the amount of generated gas (methane) was measured with a methane sensor (BCP-CH4, manufactured by BlueSens gas sensor GmbH) and a gas logger (Milligas counter, manufactured by Japan Flow Controls Co., Ltd.).

### <Test 3: 900 mV Groundwater Continuous Supply>

Anaerobic groundwater was introduced into the comb-type 18-electrode bioreactor and maintained at a flow rate of 400 mL/min. The anaerobic groundwater capacity inside the container in a steady state is 8 L. In a steady state, a voltage of 0.9 V (900 mV) was applied by a voltage application device (external power supply), and the amount of generated gas (methane) was measured with a methane sensor (BCP-CH4, manufactured by BlueSens gas sensor GmbH) and a gas logger (Milligas counter, manufactured by Japan Flow Controls Co., Ltd.).

### <Test 4: 900 mV CO₂-Dissolved Groundwater Continuous Supply>

Anaerobic groundwater was introduced into the comb-type 18-electrode bioreactor and maintained at a flow rate of 400 mL/min. The anaerobic groundwater capacity inside the container in a steady state is 8 L. In the anaerobic groundwater to be introduced into the bioreactor, carbon dioxide was further dissolved by spraying the anaerobic groundwater onto carbon dioxide (gas) using a CO₂ high-concentration dissolving apparatus (trade name: High-Concentration Oxygen Dissolving Apparatus, manufactured by TOMOE SHOKAI Co., LTD.). The dissolved carbon dioxide concentration in the anaerobic groundwater after passing through the CO₂ high-concentration dissolving apparatus was 57.1 mM. In a steady state, a voltage of 0.9 V (900 mV) was applied by a voltage application device (external power supply), and the amount of generated gas (methane) was measured with a methane sensor (BCP-CH4, manufactured by BlueSens gas sensor GmbH) and a gas logger (Milligas counter, manufactured by Japan Flow Controls Co., Ltd.).

### <Test 5: 2 V Groundwater Continuous Supply>

The amount of generated gas (methane) was measured by the same procedure as in Test 3, except that the applied voltage was changed from 0.9 V (900 mV) to 2 V

### <Test 6: 2 V CO₂-Dissolved Groundwater Continuous Supply>

The amount of generated gas (methane) was measured by the same procedure as in Test 4, except that the applied voltage was changed from 0.9 V (900 mV) to 2 V

As shown in FIG. 8, the amount of carbon dioxide (dissolved carbon dioxide concentration) contained in anaerobic groundwater is increased, and accordingly, the production rate of methane (electrogenic methane-producing rate) is remarkably improved (comparison between Test 1 and Test 2, comparison between Test 3 and Test 4, and comparison between Test 5 and Test 6). In particular, by increasing the dissolved carbon dioxide concentration from 28.5 mM to 57.1 mM using a CO₂ high-concentration dissolving apparatus, the electrogenic methane-producing rate was also increased by about 50 times (comparison between Test 3 and Test 4 and comparison between Test 5 and Test 6). Since the electrogenic methane-producing rate is not greatly increased even if the applied voltage is increased to more than 900 mV, it can be seen that a sufficient electrogenic methane-producing rate can be obtained with a less large applied voltage (1.5 V or less), and the operation can be performed at low cost.

[Test Example 4: Measurement of Electrogenic Methane-Producing Rate When Presence or Absence of Flow of Anaerobic Groundwater Is Changed] Using the comb-type 18-electrode bioreactor described in Test Example 1, the electrogenic methane-producing rate was measured when the presence or absence of a flow of anaerobic groundwater was changed.

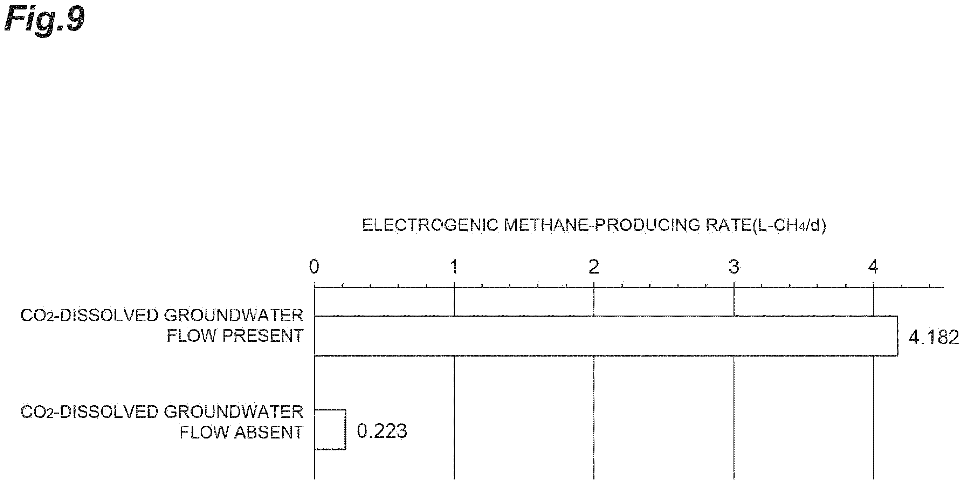

FIG. 9 is a graph showing a relationship between the presence or absence of a flow of anaerobic groundwater and an electrogenic methane-producing rate. Details of each condition in FIG. 9 are as follows.

### <CO₂-Dissolved Groundwater Flow Present>

Anaerobic groundwater was introduced into the comb-type 18-electrode bioreactor and maintained at a flow rate of 400 mL/min. The anaerobic groundwater capacity inside the container in a steady state is 8 L. In the anaerobic groundwater to be introduced into the bioreactor, carbon dioxide was further dissolved by passing the anaerobic groundwater through carbon dioxide (gas) using a CO₂ high-concentration dissolving apparatus (trade name: High-Concentration Oxygen Dissolving Apparatus, manufactured by TOMOE SHOKAI Co., LTD.). The dissolved carbon dioxide concentration in the anaerobic groundwater after passing through the CO₂ high-concentration dissolving apparatus was 57.1 mM. In a steady state, a voltage of 0.9 V (900 mV) was applied by a voltage application device (external power supply), and the amount of generated gas (methane) was measured with a methane sensor (BCP-CH4, manufactured by BlueSens gas sensor GmbH) and a gas logger (Milligas counter, manufactured by Japan Flow Controls Co., Ltd.).

### <CO₂-Dissolved Groundwater Flow Absent>

Anaerobic groundwater was introduced into the comb-type 18-electrode bioreactor, and the electrogenic methane-producing rate was measured without a flow of anaerobic groundwater (flow rate: 0 L/min). The anaerobic groundwater capacity inside the container is 8 L. In the anaerobic groundwater to be introduced into the bioreactor, carbon dioxide was further dissolved by passing the anaerobic groundwater through carbon dioxide (gas) using a CO₂ high-concentration dissolving apparatus (trade name: High-Concentration Oxygen Dissolving Apparatus, manufactured by TOMOE SHOKAI Co., LTD.). The dissolved carbon dioxide concentration in the anaerobic groundwater after passing through the CO₂ high-concentration dissolving apparatus was 57.1 mM. A voltage of 0.9 V (900 mV) was applied by a voltage application device (external power supply), and the amount of generated gas (methane) was measured with a methane sensor (BCP-CH4, manufactured by BlueSens gas sensor GmbH) and a gas logger (Milligas counter, manufactured by Japan Flow Controls Co., Ltd.).

As shown in FIG. 9, the presence of the flow of anaerobic groundwater increases the production rate of methane (electrogenic methane-producing rate) by about 20 times.

### Reference Signs List

- 10: container
- 11: inlet
- 12: outlet
- 20: plate-shaped electrode
- 20a: cathode electrode
- 20b: anode electrode
- 30: conductive wire
- 100,200: bioreactor
- 300: reaction unit
- 400: dissolving means
- 500: storage unit
- 1000: carbon dioxide recycling system

## Claims

1. A bioreactor comprising:
a container having an inlet and an outlet for a fluid; and
a plurality of plate-shaped electrodes disposed in the container, wherein
a flow path in one direction from the inlet to the outlet is formed inside the container, and
the flow path is formed such that opposing flows are adjacent to each other with the plate-shaped electrodes serving as partition walls interposed therebetween.

2. The bioreactor according to claim 1, wherein a distance between adjacent plate-shaped electrodes is 2 cm or less.

3. The bioreactor according to claim 1 or 2, wherein an anode electrode and a cathode electrode are alternately arranged in a flow path direction.

4. The bioreactor according to any one of claims 1 to 3, further comprising a voltage application device connected to the plurality of plate-shaped electrodes.

5. A carbon dioxide recycling system comprising:
a storage unit storing anaerobic groundwater;
a reaction unit converting carbon dioxide contained in the anaerobic groundwater into a resource substance by an electrochemical reaction by a microorganism; and
a transfer unit transferring the anaerobic groundwater from the storage unit to the reaction unit, wherein
the reaction unit has the bioreactor according to any one of claims 1 to 4.

6. The recycling system according to claim 5, wherein the transfer unit has a dissolving means for further dissolving carbon dioxide in the anaerobic groundwater.
